# EUROPÄISCHE PATENTSCHRIFT

(11) **EP 1 194 396 B1**
(45) Veröffentlichungstag und Bekanntmachung des Hinweises auf die Patenterteilung: **09.11.2005**
(21) Anmeldenummer: 00945733.4
(22) Anmeldetag: 09.06.2000
(51) Int. Cl.: C07C 69/75, C07C 67/303, C08K 5/12

(54) **AUSGEWÄHLTE CYCLOHEXAN-1,3- UND -1,4-DICARBONSÄUREESTER**
SELECTED CYCLOHEXANE-1,3- AND 1,4-DICARBOXYLIC ACID ESTERS
ESTERS D'ACIDE 1,3- ET 1,4-DICARBOXYLIQUE CYCLOHEXANE SELECTIONNES

(30) Priorität: 18.06.1999 DE 19927978
(43) Veröffentlichungstag der Anmeldung: 10.04.2002
(73) Patentinhaber: BASF Aktiengesellschaft, 67056 Ludwigshafen (DE)
(72) Erfinder: BRUNNER, Melanie, D-67105 Schifferstadt (DE); THIL, Lucien, D-67117 Limburgerhof (DE); BREITSCHEIDEL, Boris, D-67117 Limburgerhof (DE)
(74) Vertreter: Isenbruck, Günter
(86) Internationale Anmeldenummer: PCT/EP2000/005351
(87) Internationale Veröffentlichungsnummer: WO 2000/078704

(56) Entgegenhaltungen:
- WO-A-97/21792
- WO-A-99/32427
- US-A- 5 286 898
- DATABASE WPI Week 199512 Derwent Publications Ltd., London, GB; AN 1995-08556 XP002100461 & JP 07 011074 A (NEW JAPAN CHEM CO LTD), 13. Januar 1995 (1995-01-13)

## Beschreibung

Die vorliegende Erfindung betrifft ausgewählte Cyclohexan-1,3- und 1,4-dicarbonsäureester, sowie ihre Verwendung als Weichmacher in Kunststoffen. Ihre Herstellung mittels Hydrierung der entsprechenden Isophthalsäure- und Terephthalsäureester durch Inkontaktbringen einer oder mehrerer solcher Isophthalsäure- oder Terephthalsäureester mit einem Wasserstoff enthaltenden Gas in Gegenwart eines Makroporen aufweisenden Katalysators wird ebenfalls beschrieben.

Bislang wurden als Weichmacher in Kunststoffen, wie z.B. PVC, sehr häufig Phthalsäureester, wie z.B. Dibutyl-, Dioctyl- oder Diisononylester der Phthalsäure verwendet, wie dies z.B. aus der FR-A 23 97 131 hervorgeht. Diesen wird jedoch seit kurzer Zeit nachgesagt, daß sie gesundheitlich nicht unbedenklich sind, sodaß ihre Verwendung in Kunststoffen zur Herstellung von z.B. Kinderspielzeug immer stärker in der Kritik steht und in einigen Ländern bereits verboten ist. Im Tierversuch wurde mittlerweile gezeigt, daß Phthalate zu einer Peroxisomenproliferation führen können, welche in ursächlichem Zusammenhang mit bei Maus und Ratte in Langzeitstudien aufgetretenen Lebertumoren stehen.

Die Verwendung von einigen Cyclohexan-1,2-dicarbonsäureestern als Weichmacher ist ebenfalls aus dem Stand der Technik bekannt. So ist die Verwendung von Cyclohexandicarbonsäuredimethyl oder -diethylestem (DE-A 28 23 165) sowie von Cyclohexan-1,2-dicarbonsäuredi(2-ethylhexyl)ester (DE-A 12 63 296) als Weichmacher in Kunststoffen beschrieben.

Die PCT / EP 98 / 08346 betrifft unter anderem die Verwendung von Cyclohexanpolycarbonsäureestern als Weichmacher in Kunststoffen sowie ausgewählte neue Cyclohexan-1,2-dicarbonsäureester. Neue Cyclohexan-1,3- und -1,4-dicarbonsäurederivate werden in der PCT / EP 98 / 08346 nicht offenbart. Des weiteren werden toxikologische Eigenschaften von Cyclohexanpolycarbonsäuren und Derivaten davon in der PCT / EP 98 / 08346 mit keinem Wort erwähnt.

Die primäre Aufgabe der vorliegenden Erfindung bestand daher darin, neue Cyclohexan-1,3- und -1,4-dicarbonsäurederivate gemäß Anspruch 1 bereitzustellen, insbesondere solche, die sich zur Verwendung als Weichmacher in Kunststoff eignen, sowie deren Verwendung als Weichmacher in Kunststoffen.

Eine weitere der vorliegenden Erfindung zugrunde liegende Aufgabe lag darin, neue Cyclohexan-1,3- und -1,4-dicarbonsäurederivate für die Verwendung als Weichmacher in Kunststoffen bereitzustellen, welche nicht nur aufgrund ihrer physikalischen und stofflichen Eigenschaften für eine Verwendung als Weichmacher in Kunststoffen geeignet sind, sondern darüber hinaus auch aufgrund ihrer toxikologischen Eigenschaften im Zusammenhang mit einer Verwendung als Weichmacher in Kunststoffen als geeignet einzustufen sind.

In der US 5,286,898 und der US 5,319,129 wird Dimethylterephthalat an geträgerten Pd-Katalysatoren, die mit Ni, Pt und/oder Ru versetzt sind, bei Temperaturen ≥ 140 °C und einem Druck zwischen 50 und 170 bar zum entsprechenden Hexahydrodimethylterephthalat hydriert. In der DE-A 28 23 165 werden aromatische Carbonsäureester an geträgerten Ni-, Ru-, Rh-, und/oder Pd-Katalysatoren zu den entsprechenden cycloaliphatischen Carbonsäureestern bei 70 bis 250 °C und 30 bis 200 bar hydriert. In der US 3,027,398 wird die Hydrierung von Dimethylterephthalat an geträgerten Ru-Katalysatoren bei 110 bis 140 °C und 35 bis 105 bar beschrieben.

Die EP-A 0 603 825 betrifft ein Verfahren zur Herstellung von 1,4-Cyclohexandicarbonsäure durch Hydrierung von Terephthalsäure unter Verwendung eines geträgerten Palladium-Katalysators, wobei als Träger Aluminiumoxid, Siliciwndioxid oder Aktivkohle verwendet wird. Das dort beschriebene Verfahren ist insbesondere dadurch charakterisiert, daß die in einer ersten Stufe erhaltene 1,4-Cyclohexandicarbonsäure enthaltende Lösung mit Dampf in Kontakt gebracht wird und dadurch in dieser Lösung enthaltene Verunreinigungen extrahiert werden. Dieses Verfahren ist jedoch nur auf Säuren anwendbar, da bei der Anwendung auf Derivate, wie z.B. Ester, Anhydride, usw. die Gefahr von Hydrolyse besteht. Die Verwendung eines Makroporen aufweisenden Trägers wird in dieser Anmeldung mit keinem Wort erwähnt

Die bereits erwähnte Anmeldung PCT / EP 98 / 08346 offenbart ein Verfahren zur Hydrierung von Benzolpolycarbonsäure oder Derivaten davon, wie z.B. Estern und/oder Anhydriden, durch Inkontaktbringen einer oder mehrerer Benzolpolycarbonsäuren oder eines oder mehrerer Derivate davon mit einem Wasserstoff enthaltenden Gas in Gegenwart eines Makroporen aufweisenden Katalysators.

Demgemäß betrifft die vorliegende Erfindung ausgewählte Cyclohexan-1,3- und -1,4-dicarbonsäureester, insbesondere solche, die sich zur Verwendung als Weichmacher in Kunststoff eignen; ihre Verwendung als Weichmacher in Kunststoffen; die Herstellung dieser ausgewählten Cyclohexan-1,3- und -1,4-dicarbonsäureester mittels Hydrierung des entsprechenden Isophthalsäure- und Terephthalsäureesters oder eines Gemischs aus zwei oder mehr davon durch Inkontaktbringen einer oder mehrerer der entsprechenden Isophthal- oder Terephthalsäureester mit einem Wasserstoff enthaltenden Gas in Gegenwart eines Makroporen aufweisenden Katalysators wird ebenfalls beschrieben.

Insbesondere betrifft die vorliegende Erfindung die folgenden Cyclohexan-1,3- oder -1,4-dicarbonsäureester (vgl. Tabellen 1 und 2):
Cyclohexan-1,3-dicarbonsäure-di-pentylester, erhältlich durch Hydrierung von Dipentylisophthalat mit der Chemical Abstracts Registry-Nummer (im folgenden: CAS Nr.) 4654-16-4.
Cyclohexan-1,3-dicarbonsäure-bis(1-methylbutyl)ester, erhältlich durch Hydrierung von Bis(1-methylbutyl)isophthalat mit CAS Nr. 75151-01-8.
Cyclohexan-1,3-dicarbonsäure-diheptylester, erhältlich durch Hydrierung von Diheptylisophthalat mit CAS Nr. 4654-l7-5.
Cyclohexan-1,3-dicarbonsäure-didodecylester, erhältlich durch Hydrierung von Didodecylisophthalat mit CAS Nr. 18699-47-3.
Cyclohexan-1,3-dicarbonsäure-bis(1-methylpropyl)ester, erhältlich durch Hydrierung von Bis(1-methylpropyl)isophthalat mit der CAS Nr. 75150-99-1.
Cyclohexan-1,3-dicarbonsäure-bis(1,1-dimethylpropyl)ester, erhältlich durch Hydrierung von Bis(1,1-dimethylpropyl)isophthalat mit der CAS Nr. 117769-95-6.
Cyclohexan-1,3-dicarbonsäure-bis(2-methylbutyl)ester, erhältlich durch Hydrierung von Bis(2-methylbutyl)isophthalat mit der CAS Nr. 75151-03-0.
Cyclohexan-1,3-dicarbonsäure-bis(3-methylbutyl)ester, erhältlich durch Hydrierung von Bis(3-methylbutyl)isophthalat mit der CAS Nr. 1528-63-8.
Cyclohexan-1,3-dicarbonsäure-bis(1-ethyl-2-methylpropyl)ester, erhältlich durch Hydrierung von Bis(1-ethyl-2-methylpropyl)isophthalat mit der CAS Nr. 166391-29-3.
Cyclohexan-1,3-dicarbonsäure-bis(1-ethylbutyl)ester, erhältlich durch Hydrierung von Bis(1-ethylbutyl)isophthalat mit der CAS Nr. 166391-28-2.
Cyclohexan-1,3-dicarbonsäure-bis(1,2,2-trimethylpropyl)ester, erhältlich durch Hydrierung von Bis(1,2,2-trimethylpropyl)isophthalat mit der CAS Nr. 166391-27-1.
Cyclohexan-1,3-dicarbonsäure-bis(2-ethylbutyl)ester, erhältlich durch Hydrierung von Bis(2-ethylbutyl)isophthalat mit der CAS Nr. 166391-25-9.
Cyclohexan-1,3-dicarbonsäure-bis(4-methylpentyl)ester, erhältlich durch Hydrierung von Bis(4-methylpentyl)isophthalat mit der CAS Nr. 159375-22-1.
Cyclohexan-1,3-dicarbonsäure-bis(1,3-dimethylbutyl)ester, erhältlich durch Hydrierung von Bis(1,3-dimethylbutyl)isophthalat mit der CAS Nr. 166391-26-0.
Cyclohexan-1,3-dicarbonsäure-bis(1,1-diethylpropyl)ester, erhältlich durch Hydrierung von Bis(1,1-diethylpropyl)isophthalat mit der CAS Nr. 123095-15-8.
Cyclohexan-1,3-dicarbonsäure-bis(1-ethyl-1-methylpropyl)ester, erhältlich durch Hydrierung von Bis(1-ethyl-1-methylpropyl)isophthalat mit der CAS Nr. 145530-74-1.
Cyclohexan-1,3-dicarbonsäure-bis[2-methyl-1-(1-methylethyl)propyl]ester, erhältlich durch Hydrierung von Bis[2-methyl-1-(1-methylethyl)-propyl]isophthalat mit der CAS Nr. 166391-48-6.
Cyclohexan-1,3-dicarbonsäure-bis(2,2-dimethylhexyl)ester, erhältlich durch Hydrierung von Bis(2,2-dimethylhexyl)isophthalat mit der CAS Nr. 17673-11-9.
Cyclohexan-1,3-dicarbonsäure-bis[3-methyl-1-(2-methylpropyl)butyl]ester, erhältlich durch Hydrierung von Bis[3-methyl-1-(2-methylpropyl)butyl]-isophthalat mit der CAS Nr. 127474-92-4.
Cyclohexan-1,3-dicarbonsäure-bis(3,3,5-trimethylhexyl)ester, erhältlich durch Hydrierung von Bis(3,3,5-trimethylhexyl)isophthalat mit der CAS Nr. 208527-97-3.
Cyclohexan-1,3-dicarbonsäure-bis(2-ethyl-1,1-dimethylhexyl)ester, erhältlich durch Hydrierung von Bis(2-ethyl-1,1-dimethylhexyl)isophthalat mit der CAS Nr. 123892-24-0.
Cyclohexandicarbonsäure-1-heptyl-3-hexylester, erhältlich durch Hydrierung von 1-Heptyl-3-hexyl-isophthalat mit der CAS Nr. 166391-30-6.
Cyclohexandicarbonsäure-1-[2-ethylbutyl]-3-heptylester, erhältlich durch Hydrierung von 1-[2-Ethylbutyl]-3-heptyl-isophthalat mit der CAS Nr. 166391-41-9.
Cyclohexandicarbonsäure-1-heptyl-3-[1,2,2-trimethylpropyl]ester, erhältlich durch Hydrierung von 1-Heptyl-3-[1,2,2-trimethylpropyl]-isophthalat mit der CAS Nr. 166391-43-1.
Cyclohexandicarbonsäure-1-dimethylbutyl-3-heptylester, erhältlich durch Hydrierung von 1-Dimethylbutyl-3-heptyl-isophthalat mit der CAS Nr. 166391-42-0.
Cyclohexandicarbonsäuxe-1-[1-ethylbutyl]-3-heptylester, erhältlich durch Hydrierung von 1-[1-Ethylbutyl]-3-heptyl-isophthalat mit der CAS Nr. 166391-44-2.
Cyclohexandicarbonsäure-1-[1-ethyl-2-methylpropyl]-3-heptylester, erhältlich durch Hydrierung von 1-[1-Ethyl-2-methylpropyl]-3-heptyl-isophthalat mit der CAS Nr. 166391-45-3.
Cyclohexandicarbonsäure-1-decyl-3-hexylester, erhältlich durch Hydrierung von 1-Decyl-3-hexyl-isophthalat mit der CAS Nr. 154064-19-4.
Cyclohexandicarbonsäure-1-hexyl-3-[2-methyl-1-(1-methylethyl)propyl]ester, erhältlich durch Hydrierung von 1-Hexyl-3-[2-methyl-1-(1-methylethyl)propylisophthalat mit der CAS Nr. 166391-46-4.
Cyclohexandicarbonsäure-1-dodecyl-3-hexylester, erhältlich durch Hydrierung von 1-Dodecyl-3-hexyl-isophthalat mit der CAS Nr. 154147-75-8.
Cyclohexandicarbonsäure-1-nonyl-3-octylester, erhältlich durch Hydrierung von 1-Nonyl-3-octyl-isophthalat mit der CAS Nr. 154147-74-7.

Weitere Beispiele für Cyclohexan-1,3-dicarbonsäure-diester sind beispielsweise:
Cyclohexan-1,3-dicarbonsäure-diisobutylester, erhältlich durch Hydrierung von Diisobutylisophthalat mit der Chemical Abstracts Registry-Nummer (im Folgenden: CAS Nr.) 1528-64-9;
Cyclohexan-1,3-dicarbonsäure-di-isooctylester, erhältlich durch Hydrierung von Diisooctylisophthalat mit CAS Nr. 71850-11-8;
Cyclohexan-1,3-dicarbonsäure-di-nonylester, erhältlich durch Hydrierung von. Dinonylisophthalat mit CAS Nr. 4654-19-7;
Cyclohexan-1,3-dicarbonsäure-di-isodecylester, erhältlich durch Hydrierung von Diisodecylisophthalat mit CAS Nr. 52284-35-2;
Cyclohexan-1,3-dicarbonsäure-di-undecylester, erhältlich durch Hydrierung von Diundecylisophthalat mit CAS Nr. 18699-46-2;
Cyclohexan-1,4-dicarbonsäure-di-pentylester, erhältlich durch Hydrierung von Dipentylterephthalat mit CAS Nr. 1818-95-7.
Cyclohexan-1,4-dicarbonsäure-bis(1-methylbutyl)ester, erhältlich durch Hydrierung von Bis(1-methylbutyl)terephihalat mit CAS Nr. 75151-02-9.
Cyclohexan-1,4-dicarbonsäure-diheptylester, erhältlich durch Hydrierung von Diheptylterephthalat mit CAS Nr. 4654-25-5.
Cyclohexan-1,4-dicarbonsäure-didodecylester, erhältlich durch Hydrierung von Didodecylterephthalat mit CAS Nr. 18749-84-3.
Cyclohexan-1,4-dicarbonsäure-bis(1-methylpropyl)ester, erhältlich durch Hydrierung von Bis(1-methylpropyl)terephthalat mit der CAS Nr. 64445-74-5.
Cyclohexan-1,4-dicarbonsäure-bis(1,1-dimethylpropyl)ester, erhältlich durch Hydrierung von Bis(1,1-dimethylpropyl)terephthalat mit der CAS Nr. 117769-96-7.
Cyclohexan-1,4-dicarbonsäure-bis(2-methylbutyl)ester, erhältlich durch Hydrierung von Bis(2-methylbutyl)terephthalat mit der CAS Nr. 75151-04-1.
Cyclohexan-1,4-dicarbonsäure-bis(3-methylbutyl)ester, erhältlich durch Hydrierung von Bis(3-methylbutyl)terephthalat mit der CAS Nr. 18699-49-5.
Cyclohexan-1,4-dicarbonsäure-bis(1-ethylbutyl)ester, erhältlich durch Hydrierung von Bis(1-ethylbutyl)terephthalat mit der CAS Nr. 166391-32-8.
Cyclohexandicarbonsäure, 1-4-bis(4-methylpentyl)ester, erhältlich durch Hydrierung von Bis(4-methylpentyl)terephthalat mit der CAS Nr. 159375-21-0.
Cyclohexan-1,4-dicarbonsäure-bis[2-methyl-1-(1-methylethyl)propyl]ester, erhältlich durch Hydrierung von Bis[2-methyl-1-(1-methylethyl)propyl]-terephthalat mit der CAS Nr. 166391-33-9.
2-Methyl-Cyclohexan-1,4-dicarbonsäure-bis(2-ethylhexyl)ester, erhältlich durch Hydrierung von 2-Methyl-bis(2-ethylhexyl)terephthalat mit der CAS Nr. 51248-91-0.
Cyclohexan-1,4-dicarbonsäure-bis(1-methylheptyl)ester, erhältlich durch Hydrierung von Bis(1-methylheptyl)terephthalat mit der CAS Nr. 87321-19-5.
Cyclohexan-1,4-dicarbonsäure-bis(2-ethyl-4-methylpentyl)ester, erhältlich durch Hydrierung von Bis(2-ethyl-4-methylpentyl)terephthalat mit der CAS Nr. 59726-62-4.
Cyclohexan-1,4-dicarbonsäure-bis(2-methylheptyl)ester, erhältlich durch Hydrierung von Bis(2-methylheptyl)terephthalat mit der CAS Nr. 83789-07-5.
Cyclohexan-1,4-dicarbonsäure-bis(1,1,3,3-tetramethylbutyl)ester, erhältlich durch Hydrierung von Bis(1,1,3,3-tetramethylbutyl)terephthalat mit der CAS Nr. 90062-57-0.
Cyclohexan-1,4-dicarbonsäure-bis(7-methyloctyl)ester, erhältlich durch Hydrierung von Bis(7-methyloctyl)terephthalat mit der CAS Nr. 129951-42-4.
Cyclohexan-1,4-dicarbonsäure-bis(8-methylnonyl)ester, erhältlich durch Hydrierung von Bis(8-methylnonyl)terephthalat mit der CAS Nr. 129951-40-2.
Cyclohexandicarbonsäure-1-[8-methylnonyl]-4-octylester, erhältlich durch Hydrierung von 1-[8-Methylnonyl]-4-octyl-terephthalat mit der CAS Nr. 129951-39-9.
Cyclohexandicarbonsäure-1-decyl-4-octylester, erhältlich durch Hydrierung von 1-Decyl-4-octyl-terephthalat mit der CAS Nr. 129951-41-3.

Weitere Beispiele für Cyclohexan-1,4-dicarbonsäure-diester sind beispielsweise:
Cyclohexan-1,4-dicarbonsäure-diisobutylester, erhältlich durch Hydrierung von Diisobutylterephthalat mit der Chemical Abstracts Registry-Nummer (im Folgenden: CAS Nr.) 18699-48-4;
Cyclohexan-1,4-dicarbonsäure-di-isooctylester, erhältlich durch Hydrierung von Diisooctylterephthalat mit CAS Nr. 27937-24-2;
Cyclohexan-1,4-dicarbonsäure-di-nonylester, erhältlich durch Hydrierung von Dinonylterephthalat mit CAS Nr. 4654-27-7;
Cyclohexan-1,4-dicarbonsäure-di-isononylester, erhältlich durch Hydrierung von Diisononylterephthalat mit CAS Nr. 59802-05-0;
Cyclohexan-1,4-dicarbonsäure-di-isodecylester, erhältlich durch Hydrierung von Diisodecylterephthalat mit CAS Nr. 52174-72-8;
Cyclohexan-1,4-dicarbonsäure-di-undecylester, erhältlich durch Hydrierung von Diundecylterephthalat mit CAS Nr 111204-04-7;

Des weiteren wird beispielhaft ein Verfahren zur Herstellung der erfindungsgemäßen Cyclohexan-1,3- und -1,4-dicarbonsäurederivate oder eines Gemischs aus zwei oder mehr davon mittels Hydrierung der entsprechenden Isophthalsäure- bzw. Terephthalsäurederivate beschrieben durch Inkontaktbringen des entsprechenden Isophthalsäure- bzw. Terephthalsäurederivats oder des Gemischs aus zwei oder mehr davon mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, dadurch gekennzeichnet, daß der Träger Makroporen aufweist.

Beispielsweise wird ein Verfahren zur Hydrierung der entsprechenden Isophthalsäure- bzw. Terephthalsäureester oder eines Gemischs aus zwei oder mehr davon eingesetzt, wobei der Katalysator als Aktivmetall mindestens ein Metall der VIII. Nebengruppe des Periodensystems alleine oder zusammen mit mindestens einem Metall der I. oder VII. Nebengruppe des Periodensystems, aufgebracht auf einem Träger, umfaßt, wobei der Träger einen mittleren Porendurchmesser von mindestens 50 nm und eine BET-Oberfläche von höchstens 30 m²/g aufweist und die Menge des Aktivmetalls 0,01 bis 30 Gew.-%, bezogen auf das Gesamtgewicht des Katalysators, beträgt (Katalysator 1).

Ferner geeignet ist ein derartiges Verfahren, wobei der Katalysator als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei 10 bis 50% des Porenvolumens des Trägers von Makroporen mit einem Porendurchmesser im Bereich von 50 nm bis 10.000 nm und 50 bis 90% des Porenvolumens des Trägers von Mesoporen mit einem Porendurchmesser im Bereich von 2 bis 50 nm gebildet werden, wobei sich die Summe der Anteile der Porenvolumina zu 100% addiert (Katalysator 2).

Zur Herstellung der Ester geeignet ist darüber hinaus ein Verfahren, wie oben definiert, wobei der Katalysator (Katalysator 3) als Aktivmetall mindestens ein Metall der VIII. Nebengruppe des Periodensystems alleine oder zusammen mit mindestens einem Metall der I. oder VII. Nebengruppe des Periodensystems in einer Menge von 0,01 bis 30 Gew.-%, bezogen auf das Gesamtgewicht des Katalysators, aufgebracht auf einem Träger, umfaßt, wobei der Träger einen mittleren Porendurchmesser von mindestens 0,1 µm, und eine BET-Oberfläche von höchstens 15 m²/g aufweist. Als Träger können prinzipiell alle Träger eingesetzt werden, die Makroporen aufweisen, d.h. Träger, die ausschließlich Makroporen aufweisen sowie solche, die neben Makroporen auch Meso- und/oder Mikroporen enthalten.

Als Aktivmetall können prinzipiell alle Metalle der VIII. Nebengruppe des Periodensystems eingesetzt werden. Vorzugsweise werden als Aktivmetalle Platin, Rhodium, Palladium, Cobalt, Nickel oder Ruthenium oder ein Gemisch aus zwei oder mehr davon eingesetzt, wobei insbesondere Ruthenium als Aktivmetall verwendet wird. Unter den ebenfalls verwendbaren Metallen der I. oder VII. oder aber der I. und der VII. Nebengruppe des Periodensystems, die ebenfalls allesamt prinzipiell verwendbar sind, werden vorzugsweise Kupfer und/oder Rhenium eingesetzt.

Die Begriffe *"Makroporen"* und *"Mesoporen"* werden im Rahmen der vorliegenden Erfindung so verwendet, wie sie in *Pure Appl. Chem.,* *45**, S. 79 (1976)* definiert sind, nämlich als Poren, deren Durchmesser oberhalb von 50 nm (Makroporen) oder deren Durchmesser zwischen 2 nm und 50 nm liegt (Mesoporen).

Der Gehalt des Aktivmetalls beträgt im allgemeinen ungefähr 0,01 bis ungefähr 30 Gew.-%, vorzugsweise ungefähr 0,01 bis ungefähr 5 Gew.-% und insbesondere ungefähr 0,05 bis ungefähr 5 Gew.-%, jeweils bezogen auf das Gesamtgewicht des verwendeten Katalysators, wobei die bei den im folgenden beschriebenen, vorzugsweise eingesetzten Katalysatoren 1 bis 3 vorzugsweise verwendeten Gehalte nochmals bei der Diskussion dieser Katalysatoren einzeln angegeben sind.

Im folgenden sollen nunmehr die vorzugsweise verwendeten Katalysatoren 1 bis 3 detailliert beschrieben werden. Dabei erfolgt die Beschreibung beispielhaft unter Bezugnahme auf die Verwendung von Ruthenium als Aktivmetall. Die untenstehenden Angaben sind auch auf die anderen verwendbaren Aktivmetalle, wie hierin definiert, übertragbar.

### KATALYSATOR 1

Die beispielsweise verwendeten Katalysatoren 1 können technisch hergestellt werden durch Auftragen mindestens eines Metalls der VIII. Nebengruppe des Periodensystems und gegebenenfalls mindestens eines Metalls der I. oder VII. Nebengruppe des Periodensystems auf einem geeigneten Träger.

Die Auftragung kann durch Tränken des Trägers in wäßrigen Metallsalzlösungen, wie z.B. wäßrigen Rutheniumsalzlösungen, durch Aufsprühen entsprechender Metallsalzlösungen auf den Träger oder durch andere geeignete Verfahren erreicht werden. Als Metallsalze der I., VII. oder VIII. Nebengruppe des Periodensystems eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die neben dem Metall der VIII. Nebengruppe des Periodensystems noch weitere Metalle als Aktivmetall auf dem Träger aufgetragen enthalten, können die Metallsalze bzw. Metallsalzlösungen gleichzeitig oder nacheinander aufgebracht werden.

Die mit der Metallsalzlösung beschichteten bzw getränkten Träger werden anschließend, vorzugsweise bei Temperaturen von 100 bis 150 °C, getrocknet und wahlweise bei Temperaturen von 200 bis 600 °C, vorzugsweise von 350 bis 450 °C calciniert. Bei getrennter Auftränkung wird der Katalysator nach jedem Tränkschritt getrocknet und wahlweise calciniert, wie oben beschrieben. Die Reihenfolge, in der die Aktivkomponenten aufgetränkt werden, ist dabei frei wählbar.

Anschließend werden die beschichteten und getrockneten sowie wahlweise calcinierten Träger durch Behandlung in einem Gasstrom, der freien Wasserstoff enthält, bei Temperaturen von ungefähr 30 bis ungefähr 600 °C, vorzugsweise von ungefähr 150 bis ungefähr 450 °C aktiviert. Vorzugsweise besteht der Gasstrom aus 50 bis 100 Vol.-% H₂ und 0 bis 50 Vol.-% N₂.

Die Metallsalzlösung oder -lösungen werden in einer solchen Menge auf den oder die Träger aufgebracht, daß der Gesamtgehalt an Aktivmetall, jeweils bezogen auf das Gesamtgewicht des Katalysators, ungefähr 0,01 bis ungefähr 30 Gew.-%, vorzugsweise ungefähr 0,01 bis ungefähr 5 Gew.-%, weiter bevorzugt ungefähr 0,01 bis ungefähr 1 Gew.-%, und insbesondere ungefähr 0,05 bis ungefähr 1 Gew.-% beträgt.

Die Metalloberfläche auf dem Katalysator 1 beträgt dabei insgesamt vorzugsweise ungefähr 0,01 bis ungefähr 10 m²/g, weiter bevorzugt ungefähr 0,05 bis ungefähr 5 m²/g und insbesondere ungefähr 0,05 bis ungefähr 3 m²/g des Katalysators. Die Metalloberfläche wird mittels der von J. Lemaitre et al. in "*Characterization of Heterogeneous Catalysts"*, Hrsg. Francis Delanney, Marcel Dekker, New York 1984, S. 310 - 324, beschriebenen Chemisorptionsverfahren bestimmt.

Im beispielsweise verwendeten Katalysator 1 beträgt das Verhältnis der Oberflächen des/der Aktivmetalls/-metalle und des Katalysatorträgers vorzugsweise weniger als ungefähr 0,05, wobei der untere Grenzwert bei ungefähr 0,0005 liegt.

Die zur Herstellung der beispielsweise verwendeten Katalysatoren verwendbaren Trägermaterialien sind solche, die makroporös sind und einen mittleren Porendurchmesser von mindestens ungefähr 50 nm, vorzugsweise mindestens ungefähr 100 nm, insbesondere mindestens ungefähr 500 nm aufweisen und deren Oberfläche nach BET bei höchstens ungefähr 30 m²/g, vorzugsweise höchstens ungefähr 15 m²/g, weiter bevorzugt höchstens ungefähr 10 m²/g, insbesondere höchstens ungefähr 5 m²/g und weiter bevorzugt höchstens ungefähr 3 m²/g liegt- Der mittlere Porendurchmesser des Trägers beträgt vorzugsweise ungefähr 100 nm bis ungefähr 200 µm, weiter bevorzugt ungefähr 500 nm bis ungefähr 50 µm. Die Oberfläche des Trägers beträgt vorzugsweise ungefähr 0,2 bis ungefähr 15 m²/g, weiter bevorzugt ungefähr 0,5 bis ungefähr 10 m²/g, insbesondere ungefähr 0,5 bis ungefähr 5 m²/g und weiter bevorzugt ungefähr 0,5 bis ungefähr 3 m²/g.

Die Oberfläche des Trägers wird bestimmt nach dem BET-Verfahren durch N₂-Adsorption, insbesondere nach DIN 66131. Die Bestimmung des mittleren Porendurchmesser und der Porengrößenverteilung erfolgt durch Hg-Porosimetrie, insbesondere nach DIN 66133.

Vorzugsweise kann die Porengrößenverteilung des Trägers annähernd bimodal sein, wobei die Porendurchmesserverteilung mit Maxima bei etwa 600 nm und etwa 20 µm bei der bimodalen Verteilung eine spezielle Ausführungsform der Erfindung darstellt.

Weiter bevorzugt ist ein Träger mit einer Oberfläche von 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliciumcarbid, Aluminiumoxid, Siliciumdioxid, Titandioxid, Zirkoniumdioxid, Magnesiumoxid, Zinkoxid oder Gemische aus zwei oder mehr davon, wobei Aluminiumoxid und Zirkoniumdioxid vorzugsweise verwendet werden.

Weitere Details bezüglich Katalysator 1 bzw. zu seiner Herstellung sind der DE-A 196 24 484.6 zu entnehmen, deren diesbezüglicher Inhalt durch Bezugnahme vollständig in die vorliegende Anmeldung einbezogen wird.

### KATALYSATOR 2

Die beispielsweise verwendeten Katalysatoren 2 enthalten ein oder mehrere Metalle der VIII. Nebengruppe des Periodensystems als Aktivkomponente(n) auf einem Träger, wie hierin definiert. Bevorzugt werden Ruthenium, Palladium und/oder Rhodium als Aktivkomponente(n) verwendet.

Die beispielsweise verwendeten Katalysatoren 2 können technisch hergestellt werden durch Auftragen mindestens eines Aktivmetalls der VIII. Nebengruppe des Periodensystems, vorzugsweise Ruthenium oder Palladium und gegebenenfalls mindestens eines Metalls der I. oder VII. Nebengruppe des Periodensystems auf einem geeigneten Träger. Die Auftragung kann durch Tränken des Trägers in wäßrigen Metallsalzlösungen, wie z.B. Ruthenium- oder Palladiumsalzlösungen, durch Aufsprühen entsprechender Metallsalzlösungen auf den Träger oder durch andere geeignete Verfahren erreicht werden. Als Metallsalze zur Herstellung der Metallsalzlösungen eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, wobei die Nitrate und Nitrosylnitrate bevorzugt sind.

Bei Katalysatoren, die mehrere Aktivmetalle auf den Träger aufgetragen enthalten, können die Metallsalze bzw. Metallsalzlösungen gleichzeitig oder nacheinander aufgebracht werden.

Die mit der Metallsalzlösung beschichteten bzw, getränkten Träger werden anschließend getrocknet, wobei Temperaturen von 100 bis 150 °C bevorzugt sind. Wahlweise können diese Träger bei Temperaturen von 200 bis 600 °C, vorzugsweise von 350 bis 450 °C calciniert werden. Anschließend werden die beschichteten Träger durch Behandlung in einem Gasstrom, der freien Wasserstoff enthält, bei Temperaturen von 30 bis 600 °C, vorzugsweise von 100 bis 450 °C und insbesondere von 100 bis 300 °C aktiviert. Der Gasstrom besteht vorzugsweise aus 50 bis 100 Vol.-% H₂ und 0 bis 50 Vol.-% N₂.

Werden auf die Träger mehrere Aktivmetalle aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calciniert werden. Dabei kann die Reihenfolge, in der die Metallsalzlösung aufgetragen oder aufgetränkt wird, beliebig gewählt werden.

Die Metallsalzlösung wird in einer solchen Menge auf den/die Träger aufgebracht, daß der Gehalt an Aktivmetall 0,01 bis 30 Gew.-%, vorzugsweise 0,01 bis 10 Gew.-%, weiter bevorzugt 0,01 bis 5 Gew.-%, und insbesondere 0,3 bis 1 Gew.-%, bezogen auf das Gesamtgewicht des Katalysators, beträgt.

Die Metalloberfläche auf dem Katalysator beträgt insgesamt vorzugsweise 0,01 bis 10 m²/g, besonders bevorzugt 0,05 bis 5 m²/g und weiter bevorzugt 0,05 bis 3 m²/g des Katalysators. Die Metalloberfläche wurde durch das Chemisorptionsverfahren gemessen, wie es in J. Lemaitre et al., *"Characterization of Heterogeneous Catalysts"*, Hrsg. Francis Delanney, Marcel Dekker, New York (1984), S. 310 - 324, beschrieben ist.

Im beispielsweise verwendeten Katalysator 2 beträgt das Verhältnis der Oberflächen des mindestens einen Aktivmetalls und des Katalysatorträgers weniger als ungefähr 0,3, vorzugsweise weniger als ungefähr 0,1 und insbesondere ungefähr 0,05 oder weniger, wobei der untere Grenzwert bei ungefähr 0,0005 liegt.

Die zur Herstellung der beispielsweise verwendeten Katalysatoren 2 verwendbaren Trägermaterialien besitzen Makroporen und Mesoporen.

Dabei weisen die beispielsweise verwendbaren Träger eine Porenverteilung auf, dergemäß ungefähr 5 bis ungefähr 50%, vorzugsweise ungefähr 10 bis ungefähr 45%, weiter bevorzugt ungefähr 10 bis ungefähr 30 und insbesondere ungefähr 15 bis ungefähr 25% des Porenvolumens von Makroporen mit Porendurchmessern im Bereich von ungefähr 50 nm bis ungefähr 10.000 nm und ungefähr 50 bis ungefähr 95%, vorzugsweise ungefähr 55 bis ungefähr 90%, weiter bevorzugt ungefähr 70 bis ungefähr 90% und insbesondere ungefähr 75 bis ungefähr 85% des Porenvolumens von Mesoporen mit einem Porendurchmesser von ungefähr 2 bis ungefähr 50 nm gebildet werden, wobei sich jeweils die Summe der Anteile der Porenvolumina zu 100% addiert.

Das Gesamtporenvolumen der beispielsweise verwendeten Träger beträgt ungefähr 0,05 bis 1,5 cm³/g, vorzugsweise 0,1 bis 1,2 cm³/g und insbesondere ungefähr 0,3 bis 1,0 cm³/g. Der mittlere Porendurchmesser der erfindungsgemäß verwendeten Träger beträgt ungefähr 5 bis 20 nm, vorzugsweise ungefähr 8 bis ungefähr 15 nm und insbesondere ungefähr 9 bis ungefähr 12 nm.

Vorzugsweise beträgt die Oberfläche des Trägers ungefähr 50 bis ungefähr 500 m²/g, weiter bevorzugt ungefähr 200 bis ungefähr 350 m²/g und insbesondere ungefähr 250 bis ungefähr 300 m²/g des Trägers.

Die Oberfläche des Trägers wird nach dem BET Verfahren durch N₂-Adsorption, insbesondere nach DIN 66131, bestimmt. Die Bestimmung des mittleren Porendurchmesser und der Größenverteilung erfolgt durch Hg-Porosimetrie, insbesondere nach DIN 66133.

Obwohl prinzipiell alle bei der Katalysatorherstellung bekannten Trägermaterialien, d.h. die die oben definierte Porengrößenverteilung aufweisen, eingesetzt werden können, werden vorzugsweise Makroporen aufweisende Aktivkohle, Siliciumcarbid, Aluminiumoxid, Siliciumdioxid, Titandioxid, Zirkoniumdioxid, Magnesiumoxid, Zinkoxid oder deren Gemische, weiter bevorzugt Aluminiumoxid und Zirkoniumdioxid, eingesetzt.

Weitere Details bezüglich Katalysator 2 bzw. zu seiner Herstellung sind der DE-A 196 24 485.4 zu entnehmen, deren diesbezüglicher Inhalt durch Bezugnahme vollständig in die vorliegende Anmeldung einbezogen wird.

### KATALYSATOR 3

Die beispielsweise verwendeten Katalysatoren 3 können technisch hergestellt werden durch Auftragen eines Aktivmetalls der VIII. Nebengruppe des Periodensystems und gegebenenfalls mindestens eines Metalls der I. oder VII. Nebengruppe des Periodensystems auf einen geeigneten Träger. Die Auftragung kann durch Tränken des Trägers in wäßrigen Metallsalzlösungen, wie z.B. Rutheniumsalzlösungen, durch Aufsprühen entsprechender Metallsalzlösungen auf den Träger oder durch andere geeignete Verfahren erreicht werden. Als Rutheniumsalze zur Herstellung der Rutheniumsalzlösungen wie auch als Metallsalze der I., VII. oder VIII. Nebengruppe eignen sich die Nitrate, Nitrosylnitrate, Halogenide, Carbonate, Carboxylate, Acetylacetonate, Chlorokomplexe, Nitritokomplexe oder Aminkomplexe der entsprechenden Metalle, bevorzugt sind dabei die Nitrate und Nitrosylnitrate.

Bei Katalysatoren, die mehrere Metalle auf den Träger aufgetragen enthalten, können die Metallsalze bzw. Metallsalzlösungen gleichzeitig oder nacheinander aufgebracht werden.

Die mit der Rutheniumsalz- bzw. Metallsalzlösung beschichteten bzw. getränkten Träger werden sodann getrocknet, vorzugsweise bei Temperaturen von 100 bis 150 °C, und wahlweise bei Temperaturen von 200 bis 600 °C calciniert.

Darauffolgend werden die beschichteten Träger aktiviert durch Behandlung der beschichteten Träger in einem Gasstrom, der freien Wasserstoff enthält, bei Temperaturen von 30 bis 600 °C, vorzugsweise von 150 bis 450 °C. Der Gasstrom besteht vorzugsweise aus 50 bis 100 Vol-% H₂ und 0 bis 50 Vol-% N₂.

Werden auf die Träger neben dem Aktivmetall der VIII. Nebengruppe des Periodensystems Metalle der I. oder VII. Nebengruppe aufgetragen und erfolgt das Auftragen nacheinander, so kann der Träger nach jedem Auftragen bzw. Tränken bei Temperaturen von 100 bis 150 °C getrocknet werden und wahlweise bei Temperaturen von 200 bis 600 °C calciniert werden. Dabei kann die Reihenfolge, in der die Metallsalzlösungen aufgetragen oder aufgetränkt werden, beliebig gewählt werden.

Die Metallsalzlösung wird in einer solchen Menge auf den oder die Träger aufgebracht, daß 0,01 bis 30 Gew.-%, bezogen auf das Gesamtgewicht des Katalysators, an Aktivmetall auf den Träger aufgebracht vorliegen. Vorzugsweise beträgt diese Menge 0,2 bis 15 Gew.-%, besonders bevorzugt etwa 0,5 Gew.-%.

Die Metalloberfläche auf dem Katalysator 3 beträgt insgesamt vorzugsweise 0,01 bis 10 m²/g, besonders bevorzugt 0,05 bis 5 m²/g, insbesondere 0,05 bis 3 m² pro g des Katalysators.

Die zur Herstellung der beispielsweise verwendeten Katalysatoren 3 verwendbaren Trägermaterialien sind vorzugsweise solche, die makroporös sind und einen mittleren Porendurchmesser von mindestens 0,1 µm, vorzugsweise mindestens 0,5 µm, und eine Oberfläche von höchstens 15 m²/g aufweisen, vorzugsweise höchstens 10 m²/g, besonders bevorzugt höchstens 5 m²/g, insbesondere höchstens 3 m²/g. Bevorzugt liegt der mittlere Porendurchmesser des Trägers in einem Bereich von 0,1 bis 200 µm, insbesondere von 0,5 bis 50 µm. Bevorzugt beträgt die Oberfläche des Trägers 0,2 bis 15 m²/g, besonders bevorzugt 0,5 bis 10 m²/g, insbesondere 0,5 bis 5 m²/g, speziell 0,5 bis 3 m²/g des Trägers.

Die Oberfläche des Trägers wird bestimmt nach dem BET-Verfahren durch N₂-Adsorption, insbesondere nach DIN 66131. Die Bestimmung des mittleren Porendurchmessers und der Porengrößenverteilung erfolgte durch Hg-Porosimetrie, insbesondere nach DIN 66133. Vorzugsweise kann die Porengrößenverteilung des Trägers annähernd bimodal sein, wobei die Porendurchmesserverteilung mit Maxima bei etwa 0,6 µm und etwa 20 µm bei der bimodalen Verteilung eine spezielle Ausführungsform der Erfindung darstellt.

Besonders bevorzugt ist ein Träger mit einer Oberfläche von etwa 1,75 m²/g, der diese bimodale Verteilung des Porendurchmessers aufweist. Das Porenvolumen dieses bevorzugten Trägers beträgt vorzugsweise etwa 0,53 ml/g.

Als makroporöses Trägermaterial verwendbar sind beispielsweise Makroporen aufweisende Aktivkohle, Siliciumcarbid, Aluminiumoxid, Siliciumdioxid, Titandioxid, Zirkoniumdioxid, Magnesiumoxid, Zinkoxid oder deren Gemische. Bevorzugt sind Aluminiumoxid und Zirkoniumdioxid.

Weitere Details bezüglich Katalysator 3 bzw. zu seiner Herstellung sind der DE-A 196 04 791.9 zu entnehmen, deren diesbezüglicher Inhalt durch Bezugnahme vollständig in die vorliegende Anmeldung einbezogen wird.

### DIE VERFAHRENSFÜHRUNG - Technische Informationen

Im Rahmen eines möglichen Verfahrens zur Herstellung der erfindungsgemäßen Ester wird die Hydrierung im allgemeinen bei einer Temperatur von ungefähr 50 bis 250 °C, vorzugsweise ungefähr 70 bis 220 °C durchgeführt. Die dabei verwendeten Drücke liegen in der Regel bei oberhalb von 10 bar, vorzugsweise ungefähr 20 bis ungefähr 300 bar.

Das Verfahren kann entweder kontinuierlich oder diskontinuierlich durchgeführt werden, wobei die kontinuierliche Verfahrensdurchfiihrung bevorzugt ist.

Bei der kontinuierlichen Verfahrensführung beträgt die Menge der (des) zur Hydrierung vorgesehenen Benzoldicarbonsäureester(s) bzw. des Gemischs aus zwei oder mehr davon vorzugsweise ungefähr 0,05 bis ungefähr 3 kg pro Liter Katalysator pro Stunde, weiter bevorzugt ungefähr 0,1 bis ungefähr 1 kg pro Liter Katalysator pro Stunde.

Als Hydriergase können beliebige Gase verwendet werden, die freien Wasserstoff enthalten und keine schädlichen Mengen an Katalysatorgiften, wie beispielsweise CO, aufweisen. Beispielsweise können Reformerabgase verwendet werden. Vorzugsweise wird reiner Wasserstoff als Hydriergas verwendet.

Die Hydrierung kann in Ab- oder Anwesenheit eines Lösungs- oder Verdünnungsmittels durchgeführt werden, d.h. es ist nicht erforderlich, die Hydrierung in Lösung durchzuführen.

Vorzugsweise wird jedoch ein Lösungs- oder Verdünnungsmittel eingesetzt. Als Lösungs- oder Verdünnungsmittel kann jedes geeignete Lösungsmittel- oder Verdünnungsmittel eingesetzt werden. Die Auswahl ist dabei nicht kritisch, solange das eingesetzte Lösungs- oder Verdünnungsmittel in der Lage ist, mit dem (den) zu hydrierenden Benzoldicarbonsäureester(n) eine homogene Lösung zu bilden. Beispielsweise können die Lösungs- oder Verdünnungsmittel auch Wasser enthalten.

Beispiele geeigneter Lösungs- oder Verdünnungsmittel schließen die folgenden ein:

Geradkettige oder cyclische Ether, wie beispielsweise Tetrahydrofuran oder Dioxan, sowie aliphatische Alkohole, in denen der Alkylrest vorzugsweise 1 bis 10 Kohlenstoffatome, insbesondere 3 bis 6 Kohlenstoffatome aufweist.

Beispiele bevorzugt verwendbarer Alkohole sind i-Propanol, n-Butanol, i-Butanol und n-Hexanol.

Gemische dieser oder anderer Lösungs- oder Verdünnungsmittel können ebenfalls verwendet werden.

Die Menge des eingesetzten Lösungs- oder Verdünnungsmittels ist nicht in besonderer Weise beschränkt und kann je nach Bedarf frei gewählt werden, wobei jedoch solche Mengen bevorzugt sind, die zu einer 10 bis 70 Gew.-%igen Lösung der (des) zur Hydrierung vorgesehenen Benzoldicarbonsäureester(s) führen.

Besonders bevorzugt wird im Rahmen des möglichen Herstellungsverfahrens das bei der Hydrierung gebildete Produkt, also das entsprechende Cyclohexanderivat als Lösungsmittel eingesetzt, gegebenenfalls neben anderen Lösungs- oder Verdünnungsmitteln. In jedem Fall kann ein Teil des im Verfahren gebildeten Produkts dem (den) noch zu hydrierenden Benzoldicarbonsäurederivat(en) beigemischt werden. Bezogen auf das Gewicht der zur Hydrierung vorgesehenen Verbindung wird vorzugsweise die 1- bis 30fache, besonders bevorzugt die 5- bis 20fache, insbesondere die 5- bis 10fache Menge des Umsetzungsproduktes als Lösungs- oder Verdünnungsmittel zugemischt.

Des weiteren betrifft die vorliegende Erfindung die Verwendung der hierin offenbarten neuen Cyclohexan-1,3- bzw. -1,4-dicarbonsäurederivate als Weichmacher in Kunststoffen.

Verglichen mit den bislang hauptsächlich als Weichmacher verwendeten Phthalaten besitzen die erfindungsgemäß verwendeten Cyclohexan-1,3- und -1,4-dicarbonsäureester eine niedrigere Dichte und Viskosität und führen u.a. zu einer Verbesserung der Kälteflexibilität des Kunststoffs gegenüber der Verwendung der entsprechenden Phthalate als Weichmacher, wobei Eigenschaften wie Shore A-Härte und mechanische Eigenschaften der resultierenden Kunststoffe identisch zu denen sind, die bei Verwendung von Phthalaten resultieren. Ferner besitzen die erfindungsgemäß verwendeten Cyclohexanpolycarbonsäurederivate ein besseres Verarbeitungsverhalten im Dry-Blend und als Folge eine erhöhte Produktionsgeschwindigkeit sowie in Plastisol-Verarbeitungen Vorteile durch eine deutlich niedrigere Viskosität gegenüber den entsprechenden Phthalaten.

Neue toxikologische Befunde legen des weiteren nahe, daß die erfindungsgemäßen neuen Cyclohexan-1,3- bzw. -1,4-dicarbonsäurederivate insbesondere im Vergleich mit den bisher sehr häufig als Weichmacher verwendeten Phthalaten und Phthalsäurederivaten nicht nur im Hinblick auf physikalische und stoffliche Eigenschaften für eine Verwendung als Weichmacher geeignet sind, sondern darüber hinaus auch in toxikologischer Hinsicht als günstig zu bewerten sind.

Die eingangs erwähnte, im Zusammenhang mit Phthalaten beobachtete Entstehung von Lebertumoren in Nagern scheint über den Peroxisomenproliferator-aktivierten Rezeptor-α (PPARα; *peroxisome proliferator-activated receptor-α)* vermittelt zu werden. Die diesem Mechanismus zugrunde liegende Peroxisomenproliferation läßt sich mittels verschiedener Indikatoren, unter anderem anhand eines deutlichen Anstiegs des absoluten bzw. relativen Lebergewichtes sowie anhand eines Anstiegs bestimmter Enzymaktivitäten, etwa der spezifischen Aktivität der cyanid-insensitiven Palmitoyl-CoA Oxidase (Pal-CoA Oxidase), nachweisen.

Es ist davon auszugehen, daß die erfindungsgemäßen neuen Cyclohexan-1,3- bzw. - 1,4-dicarbonsäurederivate im Gegensatz zu verschiedenen herkömmlichen Weichmachern, insbesondere den häufig zu diesem Zweck eingesetzten Phthalaten oder Phthalsäurederivaten, keine biologisch signifikante Peroxisomenproliferation bedingen und somit nicht nur im Hinblick auf ihre physikalischen und stofflichen Eigenschaften, sondern auch in toxikologischer Hinsicht als günstig im Vergleich mit diesen herkömmlichen Weichmachern zu bewerten sind.

Im Sinne der vorliegenden Erfindung gilt eine Verbindung insbesondere dann als toxikologisch günstig, wenn im Tierversuch mit Nagern, die über eine Dauer von mindestens 14 Tagen täglich eine orale Dosis von 1000 mg / kg Körpergewicht dieser Verbindung per Schlundsonde erhalten haben, im Vergleich mit den entsprechenden unbehandelten Kontrolltieren weder ein statistisch signifikanter Anstieg des absoluten Lebergewichts oder des relativen Lebergewichts, d.h. des auf das Gesamtkörpergewicht bezogenen Lebergewichts, noch ein toxikologisch relevanter Anstieg der spezifischen Enzymaktivität der cyanid-insensitiven Palmitoyl-CoA Oxidase festgestellt wird.

Ein statistisch signifikanter Anstieg des absoluten oder relativen Lebergewichts im Sinne der vorliegenden Erfindung liegt insbesondere dann vor, wenn der statistische Anstieg des absoluten oder relativen Lebergewichts des Versuchstieres gegenüber dem des unbehandelten Kontrolltieres von mehr als 10% mittels des Dunnett-Tests bestimmt wurde (Dunnett, C.W. (1955), A multiple comparison procedure for comparing several treatments with a control, J. Am. Stat. Assoc. 50, 1096-1121; Dunnett, C.W. (1964), New tables for multiple comparisons with a control, Biometrics, 20, 482-491).

Ein toxikologisch relevanter Anstieg der spezifischen Aktivität der cyanid-insensitiven Palmitoyl-CoA Oxidase liegt dann vor, wenn die spezifische Aktivität der cyanid-insensitiven Palmitoyl-CoA Oxidase, die im Leberhomogenat eines Versuchstieres gemessen wurde, welches über die Dauer von mindestens 14 Tagen täglich eine orale Dosis von 1000 mg Prüfsubstanz / kg Körpergewicht per Schlundsonde erhalten hat, mehr als doppelt so hoch ist wie die im Leberhomogenat eines unbehandelten Kontrolltieres bestimmte spezifische Aktivität der cyanid-insensitiven Palmitoyl-CoA Oxidase.

Üblicherweise wird die spezifische Aktivität [mU/mg Protein] der cyanid-insensitiven Palmitoyl-CoA Oxidase nach Lazarow (1981), Enzymology 72, 315-319, bestimmt, wobei die Bestimmung der Proteinmenge im Leberhomogenat routinemäßig nach dem Fachmann wohlbekannten Proteinbestimmungsmethoden, etwa der Lowry-Methode, durchgeführt wird.

Darüber hinaus ist bei den hier offenbarten neuen, im Sinne der vorliegenden Erfindung toxikologisch als günstig zu bewertenden Cyclohexan-1,3- bzw. -1,4-dicarbonsäureestern zu erwarten, daß auch in bezug auf reproduktionstoxikologische Parameter relevante Verbesserungen gegenüber herkömmlichen Weichmachern, insbesonderen den sehr häufig zu diesem Zweck verwendeten Phthalaten und Phthalsäurederivaten, erreicht werden.

Demzufolge betrifft die vorliegende Erfindung insbesondere die Verwendung eines hierin offenbarten neuen Cyclohexan-1,3- bzw. -1,4-dicarbonsäurederivats oder eines Gemischs aus zwei oder mehr davon, welches im Tierversuch mit Nagern bei einer täglichen oralen Dosierung von 1000 mg / kg Körpergewicht des entsprechenden Cyclohexan-1,3- bzw. -1,4-dicarbonsäurederivats oder eines Gemischs aus zwei oder mehr davon per Schlundsonde über die Dauer von mindestens 14 Tagen im Vergleich zu unbehandelten Kontrolltieren weder zu einem signifikanten Anstieg des Lebergewichts nach der Behandlung noch zu einer Verdoppelung der im Leberhomogenat gemessenen spezifischen Aktivität der cyanid-insensitiven Palmitoyl-CoA Oxidase führt, als Weichmacher zur Herstellung toxikologisch günstig zu bewertender Kunststoffe.

Im folgenden soll nunmehr das erfindungsgemäße Verfahren anhand einiger Ausführungsbeispiele und Vergleichsbeispiele (VB) näher erläutert werden.

### BEISPIELE

### Beispiel 1: Herstellungsbeispiel

Ein meso-/makroporöser Aluminiumoxidträger in Form von 4 mm-Extrudaten, der eine BET-Oberfläche von 238 m²/g und ein Porenvolumen von 0,45 ml/g besaß, wurde mit einer wäßrigen Ruthenium-(III)-nitrat-Lösung, die eine Konzentration von 0,8 Gew.-% aufwies, getränkt. 0,15 ml/g (ungefähr 33% des Gesamtvolumens) der Poren des Trägers besaßen einen Durchmesser im Bereich von 50 nm bis 10.000 nm und 0,30 ml/g (ungefähr 67% des Gesamt Porenvolumens) der Poren des Trägers wiesen einen Porendurchmesser im Bereich von 2 bis 50 nm auf. Das während des Tränkens vom Träger aufgenommene Lösungsvolumen entsprach dabei in etwa dem Porenvolumen des verwendeten Trägers.

Anschließend wurde der mit der Ruthenium-(III)-nitrat-Lösung getränkte Träger bei 120 °C getrocknet und bei 200 °C im Wasserstoffstrom aktiviert (reduziert). Der so hergestellte Katalysator enthielt 0,05 Gew.-% Ruthenium, bezogen auf das Gewicht des Katalysators.

### Beispiel 2: Hydrierung von Terephthalsäuredi-(2-ethylhexyl)-ester

In einem 1,2 1-Druckreaktor wurden 40 g des geträgerten Ru-Katalysators gemäß Herstellungsbeispiel in einem Katalysator-Korbeinsatz vorgelegt und mit 610 g (1,56 mol) Terephthalsäuredi-(2-ethylhexyl)-ester versetzt. Die Hydrierung wurde mit reinem Wasserstoff bei einem konstanten Druck von 200 bar und einer Temperatur von 140°C durchgeführt. Es wurde so lange hydriert, bis kein Wasserstoff mehr aufgenommen wurde (3,5 h), und der Reaktor wurde anschließend entspannt. Der Umsatz an Terephthalsäuredi-(2-ethylhexyl)ester betrug 99,8%. Die Ausbeute des entsprechenden Hydrierproduktes Cyclohexan-1,4-dicarbonsäuredi-(2-ethylhexyl)-ester lag bei 97%, bezogen auf die Gesamtmenge des eingesetzten Terephthalsäuredi-(2-ethylhexyl)esters.

**Tabelle 1:**

| Edukte für die Herstellung der erfindungsgemäßen Cyclohexan-1,3-dicarbonsäureester: | |
|---|---|
| **Edukt** | **CAS Nr. des Edukts** |
| Di-isobutyl-isophthalat [Bis(2-methylpropyl)isophthalat] (VB) | 1528-64-9 |
| Di-n-pentyl-isophthalat | 4654-16-4 |
| Di-(1-methylbutyl)-isophthalat | 75151-01-8 |
| Di-n-heptyl-isophthalat | 4654-17-5 |
| Di-isooctyl-isophthalat (VB) | 71850-11-8 |
| Di-nonyl-isophthalat (VB) | 4654-19-7 |
| Di-isodecyl-isophthalat (VB) | 52284-35-2 |
| Di-undecyl-isophthalat (VB) | 18699-46-2 |
| Di-n-dodecyl-isophthalat | 18699-47-3 |
| Bis(1-methylpropyl)isophthalat | 75150-99-1 |
| Bis(1,1-dimethylpropyl)isophthalat | 117769-95-6 |
| Bis(2-methylbutyl)isophthalat | 75151-03-0 |
| Bis(3-methylbutyl)isophthalat | 1528-63-8 |
| Bis(1-ethyl-2-methylpropyl)isophthalat. | 166391-29-3 |
| Bis(1-ethylbutyl)isophthalat | 166391-28-2 |
| Bis(1,2,2-trimethylpropyl)isophthalat | 166391-27-1 |
| Bis(2-ethylbutyl)isophthalat | 166391-25-9 |
| Bis(4-methylpentyl)isophthalat | 159375-22-1 |
| Bis(1,3-dimethylbutyl)isophthalat | 166391-26-0 |
| Bis(1,1-diethylpropyl)isophthalat | 123095-15-8 |
| Bis(1-ethyl-l-methytpropyl)isophthalat | 145530-74-1 |
| Bis[2-methyl-1-(1-methylethyl)-propyl]isophthalat | 166391-48-6 |
| Bis(2,2-dimethylhexyl)isophthatat | 17673-11-9 |
| Bis[3-methyl-1-(2-methylpropyl)butyl]isophthalat | 127474-92-4 |
| Bis(3,3,5-trimethylhexyl)isophthalat | 208527-97-3 |
| Bis(2-ethyl-1,1-dimethylhexyl)isophthalat | 123892-24-0 |
| 1-Heptyl-3-hexyl-isophthalat | 166391-30-6 |
| 1-[2-Ethylbutyl]-3-heptyl-isophthalat | 166391-41-9 |
| 1-Heptyl-3-[1,2,2-trimethylpropyl]-isophthalat | 166391-43-1 |
| 1-Dimethylbutyl-3-heptyl-isophthalat | 166391-42-0 |
| 1-[1-Ethylbutyl]-3-heptyl-isophthalat | 166391-44-2 |
| 1-[1-Ethyl-2-methylpropyl]-3-heptyl-isophthalat | 166391-45-3 |
| 1-Decyl-3-hexyl-isophthalat | 154064-19-4 |
| 1-Hexyl-3-[2-methyl-1-(1-methylethyl)propyl-isophthalat | 166391-46-4 |
| 1-Dodecyl-3-hexyl-isophthalat | 154147-75-8 |
| 1-Nonyl-3-octyl-isophthalat | 154147-74-7 |

**Tabelle 2:**

| Edukte für die Herstellung der erfindungsgemäßen Cyclohexan-1,4-dicarbonsäureester: | |
|---|---|
| **Edukt** | **CAS Nr. des Edukts** |
| Di-isobutyl-terephthalat [Bis(2-methylpropyl)terephthalat] (VB) | 18699-48-4 |
| Di-n-pentyl-terephthalat | 1818-95-7 |
| Bis-(1-methylbutyl)-terephthalat | 75151-02-9 |
| Di-n-heptyl-terephthalat | 4654-25-5 |
| Di-isooctyl-terephthalat (VB) | 27937-24-2 |
| Di-nonyl-terephthalat (VB) | 4564-27-7 |
| Di-isononyl terephthalat (VB) | 59802-05-0 |
| Di-isodecyl-terephthalat (VB) | 52 174-72-8 |
| Di-undecyl-terephthalat (VB) | 111204-04-7 |
| Di-n-dodecyl-terephthalat , | 18749-84-3 |
| Bis(1-methylpropyl)terephthalat | 64445-74-5. |
| Bis(1,1-dimethylpropyl)terephthalat | 117769-96-7 |
| Bis(2-methylbutyl)terephthalat | 75151-04-1 |
| Bis(3-methylbutyl)terephthalat | 18699-49-5 |
| Bis(1-ethylbutyl)terephthalat | 166391-32-8 |
| Bis(4-methylpentyl)terephthalat | 159375-21-0 |
| Bis[2-methyl-1-(1-methylethyl)propyl]terephthalat | 166391-33-9 |
| 2-Methyl-bis(2-ethylhexyl)terephthalat | 51248-91-0 |
| Bis(1-methylheptyl)terephthalat | 87321-19-5 |
| Bis(2-ethyl-4-methylpentyl)terephthalat | 59726-62-4 |
| Bis(2-methylheptyl)terephthalat | 83789-07-5 |
| Bis(1,1,3,3-tetramethylbutyl)terephthalat | 90062-57-0 |
| Bis(7-methyloctyl)terephthalat | 129951-42-4 |
| Bis(8-methylnonyl)terephthalat | 129951-40-2 |
| 1-[8-Methytnonyl]-4-octyl-terephthalat | 129951-39-9 |
| 1-Decyl-4-octyl-terephthalat | 129951-41-3 |

## Patentansprüche

1. Cyclohexan-1,3-dicarbonsäure-di-pentylester, erhältl ich durch Hydrierung von Dipentylisophthalat mit CAS Nr. 4654-16-4;
Cyclohexan-1,3-dicarbonsäure-bis(1-methylbutyl)ester, erhältlich durch Hydrierung von Bis(1-methylbutyl)isophthalat mit CAS Nr. 75151-01-8;
Cyclohexan-1,3-dicarbonsäure-diheptylester, erhältlich durch Hydrierung von Diheptylisophthalat mit CAS Nr. 4654-17-5;
Cyclohexan-1,3-dicarbonsäure-didodecylester, erhältlich durch Hydrierung von Didodecylisophthalat mit CAS Nr. 18699-47-3;
Cyclohexan-1,3-dicarbonsäure-bis(1-methylpropyl)ester, erhältlich durch Hydrierung von Bis(1-methylpropyl)isophthalat mit der CAS Nr. 75150-99-1;
Cyclohexan-1,3-dicarbonsäurc-bis(1,1-dimethylpropyl)ester, erhältlich durch Hydrierung von Bis(1,1-dimethylpropyl)isophthalat mit der CAS Nr. 117769-95-6;
Cyclohexan-1,3-dicarbonsäure-bis(2-methylbutyl)ester, erhältlich durch Hydrierung von Bis(2-methylbutyl)isophthalat mit der CAS Nr. 75151-03-0;
Cyclohexan-1,3-dicarbonsäure-bis(3-methylbutyl)ester, erhältlich durch Hydrierung von Bis(3-methylbutyl)isophthalat mit der CAS Nr. 1528-63-8;
Cyclohexan-1,3-dicarbonsäure-bis(1-ethyl-2-methylpropyl)ester, erhältlich durch Hydrierung von Bis(1-ethyl-2-methylpropyl)isophthalat mit der CAS Nr. 166391-29-3;
Cyclohexan-1,3-dicarbonsäure-bis(1-ethylbutyl)ester, erhältlich durch Hydrierung von Bis(1-ethylbutyl)isophthalat mit der CAS Nr. 166391-28-2;
Cyclohexan-1,3-dicarbonsäure-bis(1,2,2-trimethylpropyl)ester, erhältlich durch Hydrierung von Bis(1,2,2-trimethylpropyl)isophthalat mit der CAS Nr. 166391-27-1;
Cyclohexan-1,3-dicarbonsäure-bis(2-ethylbutyl)ester, erhältlich durch Hydrierung von Bis(2-ethylbutyl)isophthalat mit der CAS Nr. 166391-25-9;
Cyclohexan-1,3-dicarbonsäure-bis(4-methylpentyl)ester, erhältlich durch Hydrierung von Bis(4-methylpentyl)isophthalat mit der CAS Nr. 159375-22-1;
Cyclohexan-1,3-dicarbonsäure-bis(1,3-dimethylbutyl)ester, erhältlich durch Hydrierung von Bis(1,3-dimethylbutyl)isophthalat mit der CAS Nr. 166391-26-0;
Cyclohexan-1,3-dicarbonsäure-bis(1,1-diethylpropyl)ester, erhältlich durch Hydrierung von Bis(1,1-diethylpropyl)isophthalat mit der CAS Nr. 123095- 15-8;
Cyclohexan-1,3-dicarbonsäure-bis(1-ethyl-1-methylpropyl)ester, erhältlich durch Hydrierung von Bis(1-ethyl-1-methylpropyl)isophthalat mit der CAS Nr. 145530-74-1;
Cyclohexan-1,3-dicarbonsäure-bis[2-methyl-1-(1-methylethyl)propyl]ester, erhältlich durch Hydrierung von Bis[2-methyl-1-(1-methylethyl)propyl]-isophthalat mit der CAS Nr. 166391-48-6;
Cyclohexan-1,3-dicarbonsäure-bis(2,2-dimethylhexyl)ester, erhältlich durch Hydrierung von Bis(2,2-dimethylhexyl)isophthalat mit der CAS Nr. 17673- 11-9;
Cyclohexan-1,3-dicarbonsäure-bis[3-methyl-1-(2-methylpropyl)butyl]ester, erhältlich durch Hydrierung von Bis[3-methyl-1-(2-methylpropyl)butyl]-isophthalat mit der CAS Nr. 127474-92-4;
Cyclohexan-1,3-dicarbonsäure-bis(3,3,5-trimethylhexyl)ester, erhältlich durch Hydrierung von Bis(3,3,5-trimethylhexyl)isophthalat mit der CAS Nr. 208527-97-3;
Cyclohexan-1,3-dicarbonsäure-bis(2-ethyl-1,1-dimethylhexyl)ester, erhältlich durch Hydrierung von Bis(2-ethyl-1,1-dimethylhexyl)isophthalat mit der CAS Nr. 123892-24-0;
Cyclohexandicarbonsäure-1-heptyl-3-hexylester, erhältlich durch Hydrierung von 1-Heptyl-3-hexyl-isophthalat mit der CAS Nr. 166391-30-6;
Cyclohexandicarbonsäure-1-[2-ethylbutyl]-3-heptylester, erhältlich durch Hydrierung von 1-[2-Ethylbutyl]-3-heptyl-isophthalat mit der CAS Nr. 166391-41-9;
Cyclohexandicarbonsäure-1-heptyl-3-[1,2,2-trimethylpropyl]ester, erhältlich durch Hydrierung von 1-Heptyl-3-[1,2,2-trimethylpropyl]-isophthalat mit der CAS Nr. 166391-43-1;
Cyclohexandicarbonsäure-1-dimethylbutyl-3-heptylester, erhältlich durch Hydrierung von 1-Dimethylbutyl-3-heptyl-isophthalat mit der CAS Nr. 166391-42-0;
Cyclohexandicarbonsäure-1-[1-ethylbutyl]-3-heptylester, erhältlich durch Hydrierung von 1-[1-Ethylbutyl]-3-heptyl-isophthalat mit der CAS Nr. 166391-44-2;
Cyclohexandicarbonsäure-1-[1-ethyl-2-methylpropyl]-3-heptylester, erhältlich durch Hydrierung von 1-[1-Ethyl-2-methylpropyl]-3-heptyl-isophthalat mit der CAS Nr. 166391-45-3;
Cyclohexandicarbonsäure-1-decyl-3-hexylester, erhältlich durch Hydrierung von 1-Decyl-3-hexyl-isophthalat mit der CAS Nr. 154064-19-4;
Cyclohexandicarbonsäure-1-hexyl-3-[2-methyl-1-(1-methylethyl)propyl]-ester, erhältlich durch Hydrierung von 1-Hexyl-3-[2-methyl-1-(1-methylethyl)propyl]isophthalat mit der CAS Nr. 166391-46-4;
Cyclohexandicarbonsäure-1-dodecyl-3-hexylester, erhältlich durch Hydrierung von 1-Dodecyl-3-hexyl-isophthalat mit der CAS Nr. 154147-75-8;
Cyclohexandicarbonsäure-1-nonyl-3-octylester, erhältlich durch Hydrierung von 1-Nonyl-3-octyl-isophthalat mit der CAS Nr. 154147-74-7;
Cyclohexan-1,4-dicarbonsäure-di-pentylester, erhältlich durch Hydrierung von Dipentylterephthalat mit CAS Nr. 1818-95-7;
Cyclohexan-1,4-dicarbonsäure-bis(1-methylbutyl)ester, erhältlich durch Hydrierung von Bis(1-methylbutyl)terephthalat mit CAS Nr. 75151-02-9;
Cyclohexan-1,4-dicarbonsäure-diheptylester, erhältlich durch Hydrierung von Diheptylterephthalat mit CAS Nr. 4654-25-5;
Cyclohexan-1,4-dicarbonsäure-didodecylester, erhältlich durch Hydrierung von Didodecylterephthalat mit CAS Nr. 18749-84-3;
Cyclohexan-1,4-dicarbonsäure-bis(1-methylpropyl)ester, erhältlich durch Hydrierung von Bis(1-methylpropyl)terephthalat mit der CAS Nr. 64445-74-5;
Cyclohexan-1,4-dicarbonsäure-bis(1,1-dimethylpropyl)ester, erhältlich durch Hydrierung von Bis(1,1-dimethylpropyl)terephthalat mit der CAS Nr. 117769-96-7;
Cyclohexan-1,4-dicarbonsäure-bis(2-methylbutyl)ester, erhältlich durch Hydrierung von Bis(2-methylbutyl)terephthalat mit der CAS Nr. 75151-04-1;
Cyclohexan-1,4-dicarbonsäure-bis(3-methylbutyl)ester, erhältlich durch Hydrierung von Bis(3-methylbutyl)terephthalat mit der CAS Nr. 18699-49-5;
Cyclohexan-1,4-dicarbonsäure-bis(1-ethylbutyl)ester, erhältlich durch Hydrierung von Bis(1-ethylbutyl)terephthalat mit der CAS Nr. 166391-32-8;
Cyclohexandicarbonsäure-1,4-bis(4-methylpentyl)ester, erhältlich durch Hydrierung von Bis(4-methylpentyl)terephthalat mit der CAS Nr. 159375-21-0;
Cyclohexan-1,4-dicarbonsäure-bis[2-methyl-1-(1-methylethyl)propyl]ester, erhältlich durch Hydrierung von Bis[2-methyl-1-(1-methylethyl)-propyl]terephthalat mit der CAS Nr. 166391-33-9;
2-Methyl-Cyclohexan-1,4-dicarbonsäure-bis(2-ethythexyl)ester, erhältlich durch Hydrierung von 2-Methyl-bis(2-ethylhexyl)terephthalat mit der CAS Nr. 51248-91-0;
Cyclohexan-1,4-dicarbonsäure-bis(1-methylheptyl)ester, erhältlich durch Hydrierung von Bis(1-methylheptyl)terephthalat mit der CAS Nr. 87321-19-5;
Cyclohexan-1,4-dicarbonsäure-bis(2-ethyl-4-methylpentyl)ester, erhältlich durch Hydrierung von Bis(2-ethyl-4-methylpentyl)terephthalat mit der CAS Nr. 59726-62-4;
Cyclohexan-1,4-dicarbonsäure-bis(2-methylheptyl)ester, erhältlich durch Hydrierung von Bis(2-methylheptyl)terephthalat mit der CAS Nr. 83789-07-5;
Cyclohexan-1,4-dicarbonsäure-bis(1,1,3,3-tetramethylbutyl)ester, erhältlich durch Hydrierung von Bis(1,1,3,3-tetramethylbutyl)terephthalat mit der CAS Nr. 90062-57-0;
Cyclohexan-1,4-dicarbonsäure-bis(7-methyloctyl)ester, erhältlich durch Hydrierung von Bis(7-methyloctyl)terephthalat mit der CAS Nr. 129951-42-4;
Cyclohexan-1,4-dicarbonsäure-bis(8-methylnonyl)ester, erhältlich durch Hydrierung von Bis(8-methylnonyl)terephthalat mit der CAS Nr. 129951-40-2;
Cyclohexandicarbonsäure-1-[8-methylnonyl]-4-octylester, erhältlich durch Hydrierung von 1-[8-Methylnonyl]-4-octyl-terephthalat mit der CAS Nr. 129951-39-9;
Cyclohexandicarbonsäure-1-decyl-4-octylester, erhältlich durch Hydrierung von 1-Decyl-4-octyl-terephthalat mit der CAS Nr. 129951-41-3.

2. Verwendung eines Cyclohexan-1,3- oder -1,4-dicarbonsäureesters gemäß Anspruch 1 oder eines Gemisches aus zwei oder mehr davon als Weichmacher in Kunststoffen.

3. Verwendung eines Cyclohexan-1,3- bzw. -1,4-dicarbonsäureesters gemäß Anspruch 1 oder eines Gemischs aus zwei oder mehr davon, welcher/welches im Tierversuch mit Nagern bei einer täglichen oralen Dosierung von 1000 mg/kg Körpergewicht des entsprechenden Cyclohexan-1,3- bzw. -1,4-dicarbonsäureesters oder des Gemischs aus zwei oder mehr davon per Schlundsonde über die Dauer von mindestens 14 Tagen im Vergleich zu unbehandelten Kontrolltieren weder zu einem signifikanten Anstieg des Lebergewichts nach der Behandlung noch zu einer Verdoppelung der im Leberhomogenat gemessenen spezifischen Aktivität der cyanid-insensitiven Palmitoyl-CoA Oxidase führt, als Weichmacher zur Herstellung toxikologisch günstig zu bewertender Kunststoffe.

## Claims

1. Cyclohexane-1,3-dicarboxylic acid dipentyl ester obtainable by hydrogenating dipentyl isophthalate with CAS No. 4654-16-4;
Cyclohexane-1,3-dicarboxylic acid bis(1-methylbutyl) ester obtainable by hydrogenating bis(1-methylbutyl) isophthalate with CAS No. 75151-01-8;
Cyclohexane-1,3-dicarboxylic acid diheptyl ester obtainable by hydrogenating diheptyl isophthalate with CAS No. 4654-17-5;
Cyclohexane-1,3-dicarboxylic acid didodecyl ester obtainable by hydrogenating didodecyl isophthalate with CAS No. 18699-47-3;
Cyclohexane-1,3-dicarboxylic acid bis(1-methylpropyl) ester obtainable by hydrogenating bis(1-methylpropyl) isophthalate with CAS No. 75150-99-1;
Cyclohexane-1,3-dicarboxylic acid bis(1,1-dimethylpropyl) ester obtainable by hydrogenating bis(1,1-dimethylpropyl) isophthalate with CAS No. 117769-95-6;
Cyclohexane-1,3-dicarboxylic acid bis(2-methylbutyl) ester obtainable by hydrogenating bis(2-methylbutyl) isophthalate with CAS No. 75151-03-0;
Cyclohexane-1,3-dicarboxylic acid bis(3-methylbutyl) ester obtainable by hydrogenating bis(3-methylbutyl) isophthalate with CAS No. 1528-63-8;
Cyclohexane-1,3-dicarboxylic acid bis(l-ethyl-2-methylpropyl) ester obtainable by hydrogenating bis(l-ethyl-2-methylpropyl) isophthalate with CAS No. 166391-29-3;
Cyclohexane-1,3-dicarboxylic acid bis(1-ethylbutyl) ester obtainable by hydrogenating bis(1-ethylbutyl) isophthalate with CAS No. 166391-28-2;
Cyclohexane-1,3-dicarboxylic acid bis(1,2,2-trimethylpropyl) ester obtainable by hydrogenating bis(1,2,2-trimethylpropyl) isophthalate with CAS No. 166391-27-1;
Cyclohexane-1,3-dicarboxylic acid bis(2-ethylbutyl) ester obtainable by hydrogenating bis(2-ethylbutyl) isophthalate with CAS No. 166391-25-9;
Cyclohexane-1,3-dicarboxylic acid bis(4-methylpentyl) ester obtainable by hydrogenating bis(4-methylpentyl) isophthalate with CAS No. 159375-22-1;
Cyclohexane-1,3-dicarboxylic acid bis(1,3-dimethylbutyl) ester obtainable by hydrogenating bis(1,3-dimethylbutyl) isophthalate with CAS No. 166391-26-0;
Cyclohexane-1,3-dicarboxylic acid bis(1,1-diethylpropyl) ester obtainable by hydrogenating bis(1,1-diethylpropyl) isophthalate with CAS No. 123095-15-8;
Cyclohexane-1,3-dicarboxylic acid bis(1-ethyl-1-methylpropyl) ester obtainable by hydrogenating bis(1-ethyl-1-methylpropyl) isophthalate with CAS No. 145530-74-1;
Cyclohexane-1,3-dicarboxylic acid bis[2-methyl-1-(1-methylethyl)propyl] ester obtainable by hydrogenating bis[2-methyl-1-(1-methylethyl)propyl] isophthalate with CAS No. 166391-48-6;
Cyclohexane-1,3-dicarboxylic acid bis(2,2-dimethylhexyl) ester obtainable by hydrogenating bis(2,2-dimethylhexyl) isophthalate with CAS No. 17673-11-9;
Cyclohexane-1,3-dicarboxylic acid bis[3-methyl-1-(2-methylpropyl)butyl] ester obtainable by hydrogenating bis[3-methyl-1-(2-methylpropyl)butyl] isophthalate with CAS No. 127474-92-4;
Cyclohexane-1,3-dicarboxylic acid bis(3,3,5-trimethylhexyl) ester obtainable by hydrogenating bis(3,3,5-trimethylhexyl) isophthalate with CAS No. 208527-97-3;
Cyclohexane-1,3-dicarboxylic acid bis(2-ethyl-1,1-dimethylhexyl) ester obtainable by hydrogenating bis(2-ethyl-1,1-dimethylhexyl) isophthalate with CAS No. 123892-24-0;
Cyclohexanedicarboxylic acid 1-heptyl-3-hexyl ester obtainable by hydrogenating 1-heptyl-3-hexyl isophthalate with CAS No. 166391-30-6;
Cyclohexanedicarboxylic acid 1-[2-ethylbutyl]-3-heptyl ester obtainable by hydrogenating 1-[2-ethylbutyl]-3-heptyl isophthalate with CAS No. 166391-41-9;
Cyclohexanedicarboxylic acid 1-heptyl-3-[1,2,2-trimethylpropyl] ester obtainable by hydrogenating 1-heptyl-3-[1,2,2-trimethylpropyl] isophthalate with CAS No. 166391-43-1;
Cyclohexanedicarboxylic acid I-dimethylbutyl-3-heptyl ester obtainable by hydrogenating l-dimethylbutyl-3-heptyl isophthalate with CAS No. 166391-42-0;
Cyclohexanedicarboxylic acid 1-[1-ethylbutyl]-3-heptyl ester obtainable by hydrogenating 1-[1-ethylbutyl]-3-heptyl isophthalate with CAS No. 166391-44-2;
Cyclohexanedicarboxylic acid 1-[1-ethyl-2-methylpropyl]-3-heptyl ester obtainable by hydrogenating 1-[1-ethyl-2-methylpropyl]-3-heptyl isophthalate with CAS No. 166391-45-3;
Cyclohexanedicarboxylic acid 1-decyl-3-hexyl ester obtainable by hydrogenating 1-decyl-3-hexyl isophthalate with CAS No. 154064-19-4;
Cyclohexanedicarboxylic acid 1-hexyl-3-[2-methyl-1-(1-methylethyl)propyl] ester obtainable by hydrogenating 1-hexyl-3-[2-methyl-1-(1-methylethyl)propyl] isophthalate with CAS No. 166391-46-4;
Cyclohexanedicarboxylic acid 1-dodecyl-3-hexyl ester obtainable by hydrogenating 1-dodecyl-3-hexyl isophthalate with CAS No. 154147-75-8;
Cyclohexanedicarboxylic acid 1-nonyl-3-octyl ester obtainable by hydrogenating 1-nonyl-3-octyl isophthalate with CAS No. 154147-74-7;
Cyclohexane-1,4-dicarboxylic acid dipentyl ester obtainable by hydrogenating dipentyl terephthalate with CAS No. 1818-95-7;
Cyclohexane-1,4-dicarboxylic acid bis(1-methylbutyl) ester obtainable by hydrogenating bis(1-methylbutyl) terephthalate with CAS No. 75151-02-9;
Cyclohexane-1,4-dicarboxylic acid diheptyl ester obtainable by hydrogenating diheptyl terephthalate with CAS No. 4654-25-5;
Cyclohexane-1,4-dicarboxylic acid didodecyl ester obtainable by hydrogenating didodecyl terephthalate with CAS No. 18749-84-3;
Cyclohexane-1,4-dicarboxylic acid bis(1-methylpropyl) ester obtainable by hydrogenating bis(1-methylpropyl) terephthalate with CAS No. 64445-74-5;
Cyclohexane-1,4-dicarboxylic acid bis(1,1-dimethylpropyl) ester obtainable by hydrogenating bis(1,1-dimethylpropyl) terephthalate with CAS No. 117769-96-7;
Cyclohexane-1,4-dicarboxylic acid bis(2-methylbutyl) ester obtainable by hydrogenating bis(2-methylbutyl) terephthalate with CAS No. 75151-04-1;
Cyclohexane-1,4-dicarboxylic acid bis(3-methylbutyl) ester obtainable by hydrogenating bis(3-methylbutyl) terephthalate with CAS No. 18699-49-5;
Cyclohexane-1,4-dicarboxylic acid bis(1-ethylbutyl) ester obtainable by hydrogenating bis(1-ethylbutyl) terephthalate with CAS No. 166391-32-8;
Cyclohexane-1,4-dicarboxylic acid bis(4-methylpentyl) ester obtainable by hydrogenating bis(4-methylpentyl) terephthalate with CAS No. 159375-21-0;
Cyclohexane-1,4-dicarboxylic acid bis[2-methyl-1-(1-methylethyl)propyl] ester obtainable by hydrogenating bis[2-methyl-1-(1-methylethyl)propyl] terephthalate with CAS No. 166391-33-9;
2-Methylcyclohexane-1,4-dicarboxylic acid bis(2-ethylhexyl) ester obtainable by hydrogenating bis(2-ethylhexyl) 2-methylterephthalate with CAS No. 51248-91-0;
Cyclohexane-1,4-dicarboxylic acid bis(1-methylheptyl) ester obtainable by hydrogenating bis(1-methylheptyl) terephthalate with CAS No. 87321-19-5;
Cyclohexane-1,4-dicarboxylic acid bis(2-ethyl-4-methylpentyl) ester obtainable by hydrogenating bis(2-ethyl-4-methylpentyl) terephthalate with CAS No. 59726-62-4;
Cyclohexane-1,4-dicarboxylic acid bis(2-methylheptyl) ester obtainable by hydrogenating bis(2-methylheptyl) terephthalate with CAS No. 83789-07-5;
Cyclohexane-1,4-dicarboxylic acid bis(1,1,3,3-tetramethylbutyl) ester obtainable by hydrogenating bis(1,1,3,3-tetramethylbutyl) terephthalate with CAS No. 90062-57-0;
Cyclohexane-1,4-dicarboxylic acid bis(7-methyloctyl) ester obtainable by hydrogenating bis(7-methyloctyl) terephthalate with CAS No. 129951-42-4;
Cyclohexane-1,4-dicarboxylic acid bis(8-methylnonyl) ester obtainable by hydrogenating bis(8-methylnonyl) terephthalate with CAS No. 129951-40-2;
Cyclohexanedicarboxylic acid 1-[8-methylnonyl]-4-octyl ester obtainable by hydrogenating 1-[8-methylnonyl]-4-octyl terephthalate with CAS No. 129951-39-9;
Cyclohexanedicarboxylic acid 1-decyl-4-octyl ester obtainable by hydrogenating 1-decyl-4-octyl terephthalate with CAS No. 129951-41-3.

2. The use of a cyclohexane-1,3- or -1,4-dicarboxylic acid ester according to claim 1 or of a mixture made from two or more of these as a plasticizer in plastics.

3. The use, as a plasticizer for preparing plastics advantageous from a toxicological point of view, of a cyclohexane-1,3- or -1,4-dicarboxylic acid ester according to claim 1 or of a mixture made from two or more of these which, when tested on rodents at a daily oral dose of 1000 mg/kg of body weight of the appropriate cyclohexane-1,3- or -1,4-dicarboxylic acid ester or of the mixture made from two or more of these, via a stomach tube, over a period of at least 14 days, gives no significant rise in the liver weight after the treatment and does not give a doubling of the specific activity, measured in the liver homogenate, of the cyanide-insensitive palmitoyl-CoA oxidase when comparison is made with untreated control animals.

## Revendications

1. Cyclohexane-1,3-dicarboxylate de dipentyle, que l'on peut obtenir par hydrogénation d'isophtalate de dipentyle portant le numéro CAS 4654-16-4;
cyclohexane-1,3-dicarboxylate de bis(1-méthylbutyle), que l'on peut obtenir par hydrogénation d'isophtalate de bis(1-méthylbutyle) portant le numéro CAS 75151-01-8;
cyclohexane-1,3-dicarboxylate de diheptyle, que l'on peut obtenir par hydrogénation d'isophtalate de diheptyle portant le numéro CAS 4654-17-5;
cyclohexane-1,3-dicarboxylate de didodécyle, que l'on peut obtenir par hydrogénation d'isophtalate de didodécyle portant le numéro CAS 18699-47-3;
cyclohexane-1,3-dicarboxylate de bis(1-méthylpropyle), que l'on peut obtenir par hydrogénation d'isophtalate de bis(1-méthylpropyle) portant le numéro CAS 75150-99-1;
cyclohexane-1,3-dicarboxylate de bis(1,1-diméthylpropyle), que l'on peut obtenir par hydrogénation d'isophtalate de bis(1,1-diméthylpropyle) portant le numéro CAS 117769-95-6;
cyclohexane-1,3-dicarboxylate de bis(2-méthylbutyle), que l'on peut obtenir par hydrogénation d'isophtalate de bis(2-méthylbutyle) portant le numéro CAS 75151-03-0;
cyclohexane-1,3-dicarboxylate de bis(3-méthylbutyle), que l'on peut obtenir par hydrogénation d'isophtalate de bis(3-méthylbutyle) portant le numéro CAS 1528-63-8;
cyclohexane-1,3-dicarboxylate de bis(1-éthyl-2-méthylpropyle), que l'on peut obtenir par hydrogénation d'isophtalate de bis(1-éthyl-2-méthylpropyle) portant le numéro CAS 166391-29-3;
cyclohexane-1,3-dicarboxylate de bis(1-éthylbutyle), que l'on peut obtenir par hydrogénation d'isophtalate de bis(1-éthylbutyle) portant le numéro CAS 166391-28-2;
cyclohexane-1,3-dicarboxylate de bis(1,2,2-triméthylpropyle), que l'on peut obtenir par hydrogénation d'isophtalate de bis(1,2,2-triméthylpropyle) portant le numéro CAS 166391-27-1;
cyclohexane-1,3-dicarboxylate de bis(2-éthylbutyle), que l'on peut obtenir par hydrogénation d'isophtalate de bis(2-éthylbutyle) portant le numéro CAS 166391-25-9;
cyclohexane-1,3-dicarboxylate de bis(4-méthylpentyle), que l'on peut obtenir par hydrogénation d'isophtalate de bis(4-méthylpentyle) portant le numéro CAS 159375-22-1;
cyclohexane-1,3-dicarboxylate de bis(1,3-diméthylbutyle), que l'on peut obtenir par hydrogénation d'isophtalate de bis(1,3-diméthylbutyle) portant le numéro CAS 166391-26-0;
cyclohexane-1,3-dicarboxylate de bis(1,1-diéthylpropyle), que l'on peut obtenir par hydrogénation d'isophtalate de bis(1,1-diéthylpropyle) portant le numéro CAS 123095-15-8;
cyclohexane-1,3-dicarboxylate de bis(1-éthyl-1-méthylpropyle), que l'on peut obtenir par hydrogénation d'isophtalate de bis(1-éthyl-1-méthylpropyle) portant le numéro CAS 145530-74-1;
cyclohexane-1,3-dicarboxylate de bis[2-méthyl-1-(1-méthyléthyl)propyle], que l'on peut obtenir par hydrogénation d'isophtalate de bis[2-méthyl-1-(1-méthyléthyl)propyle] portant le numéro CAS 166391-48-6;
cyclohexane-1,3-dicarboxylate de bis(2,2-diméthylhexyle), que l'on peut obtenir par hydrogénation d'isophtalate de bis(2,2-diméthylhexyle) portant le numéro CAS 17673-11-9;
cyclohexane-1,3-dicarboxylate de bis[3-méthyl-1-(2-méthylpropyl)butyle], que l'on peut obtenir par hydrogénation d'isophtalate de bis[3-méthyl-1-(2-méthylpropyl)butyle] portant le numéro CAS 127474-92-4;
cyclohexane-1,3-dicarboxylate de bis(3,3,5-triméthylhexyle), que l'on peut obtenir par hydrogénation d'isophtalate de bis(3,3,5-triméthylhexyle) portant le numéro CAS 208527-97-3;
cyclohexane-1,3-dicarboxylate de bis(2-éthyl-1,1-diméthylhexyle), que l'on peut obtenir par hydrogénation d'isophtalate de bis(2-éthyl-1,1-diméthylhexyle) portant le numéro CAS 123892-24-0;
cyclohexanedicarboxylate de 1-heptyl-3-hexyle, que l'on peut obtenir par hydrogénation d'isophtalate de 1-heptyl-3-hexyle portant le numéro CAS 166391-30-6;
cyclohexanedicarboxylate de 1-[2-éthylbutyl]-3-heptyle, que l'on peut obtenir par hydrogénation d'isophtalate de 1-[2-éthylbutyl]-3-heptyle portant le numéro CAS 166391-41-9;
cyclohexanedicarboxylate de 1-heptyl-3-[1,2,2-triméthylpropyle], que l'on peut obtenir par hydrogénation d'isophtalate de 1-heptyl-3-[1,2,2-triméthylpropyle] portant le numéro CAS 166391-43-1;
cyclohexanedicarboxylate de 1-diméthylbutyl-3-heptyle, que l'on peut obtenir par hydrogénation d'isophtalate de 1-diméthylbutyl-3-heptyle portant le numéro CAS 166391-42-0;
cyclohexanedicarboxylate de 1-[1-éthylbutyl]-3-heptyle, que l'on peut obtenir par hydrogénation d'isophtalate de 1-[1-éthylbutyl]-3-heptyle portant le numéro CAS 166391-44-2;
cyclohexanedicarboxylate de 1-[1-éthyl-2-méthylpropyl]-3-heptyle, que l'on peut obtenir par hydrogénation d'isophtalate de 1-[1-éthyl-2-méthylpropyl]-3-heptyle portant le numéro CAS 166391-45-3;
cyclohexanedicarboxylate de 1-décyl-3-hexyle, que l'on peut obtenir par hydrogénation d'isophtalate de 1-décyl-3-hexyle portant le numéro CAS 154064-19-4;
cyclohexanedicarboxylate de 1-hexyl-3-[2-méthyl-1-(1-méthyléthyl)propyle], que l'on peut obtenir par hydrogénation d'isophtalate de 1-hexyl-3-[2-méthyl-1-(1-méthyléthyl)propyle] portant le numéro CAS 166391-46-4;
cyclohexanedicarboxylate de 1-dodécyl-3-hexyle, que l'on peut obtenir par hydrogénation d'isophtalate de 1-dodécyl-3-hexyle portant le numéro CAS 154147-75-8;
cyclohexanedicarboxylate de 1-nonyl-3-octyle, que l'on peut obtenir par hydrogénation d'isophtalate de 1-nonyl-3-octyle portant le numéro CAS 154147-74-7;
cyclohexane-1,4-dicarboxylate de dipentyle, que l'on peut obtenir par hydrogénation de téréphtalate de dipentyle portant le numéro CAS 1818-95-7;
cyclohexane-1,4-dicarboxylate de bis(1-méthylbutyle), que l'on peut obtenir par hydrogénation de téréphtalate de bis(1-méthylbutyle) portant le numéro CAS 75151-02-9;
cyclohexane-1,4-dicarboxylate de diheptyle, que l'on peut obtenir par hydrogénation de téréphtalate de diheptyle portant le numéro CAS 4654-25-5;
cyclohexane-1,4-dicarboxylate de didodécyle, que l'on peut obtenir par hydrogénation de téréphtalate de didodécyle portant le numéro CAS 18749-84-3;
cyclohexane-1,4-dicarboxylate de bis(1-méthylpropyle), que l'on peut obtenir par hydrogénation de téréphtalate de bis(1-méthylpropyle) portant le numéro CAS 64445-74-5;
cyclohexane-1,4-dicarboxylate de bis(1,1-diméthylpropyle), que l'on peut obtenir par hydrogénation de téréphtalate de bis(1,1-diméthylpropyle) portant le numéro CAS 117769-96-7;
cyclohexane-1,4-dicarboxylate de bis(2-méthylbutyle), que l'on peut obtenir par hydrogénation de téréphtalate de bis(2-méthylbutyle) portant le numéro CAS 75151-04-1;
cyclohexane-1,4-dicarboxylate de bis(3-méthylbutyle), que l'on peut obtenir par hydrogénation de téréphtalate de bis(3-méthylbutyle) portant le numéro CAS 18699-49-5;
cyclohexane-1,4-dicarboxylate de bis(1-éthylbutyle), que l'on peut obtenir par hydrogénation de téréphtalate de bis(1-éthylbutyle) portant le numéro CAS 166391-32-8;
cyclohexanedicarboxylate de 1,4-bis(4-méthylpentyle), que l'on peut obtenir par hydrogénation de téréphtalate de bis (4-méthylpentyle) portant le numéro CAS 159375-21-0;
cyclohexane-1,4-dicarboxylate de bis[2-méthyl-1-(1-méthyléthyl)propyle], que l'on peut obtenir par hydrogénation de téréphtalate de bis[2-méthyl-1-(1-méthyléthyl)propyle] portant le numéro CAS 166391-33-9;
2-méthylcyclohexane-1,4-dicarboxylate de bis(2-éthylhexyle), que l'on peut obtenir par hydrogénation de téréphtalate de 2-méthyl-bis(2-éthylhexyle) portant le numéro CAS 51248-91-0;
cyclohexane-1,4-dicarboxylate de bis(1-méthylheptyle), que l'on peut obtenir par hydrogénation de téréphtalate de bis(1-méthylheptyle) portant le numéro CAS 87321-19-5;
cyclohexane-1,4-dicarboxylate de bis(2-éthyl-4-méthylpentyle), que l'on peut obtenir par hydrogénation de téréphtalate de bis(2-éthyl-4-méthylpentyle) portant le numéro CAS 59726-62-4;
cyclohexane-1,4-dicarboxylate de bis(2-méthylheptyle), que l'on peut obtenir par hydrogénation de téréphtalate de bis(2-méthylheptyle) portant le numéro CAS 83789-07-5;
cyclohexane-1,4-dicarboxylate de bis(1,1,3,3-tétraméthylbutyle), que l'on peut obtenir par hydrogénation de téréphtalate de bis(1,1,3,3-tétraméthylbutyle) portant le numéro CAS 90062-57-0;
cyclohexane-1,4-dicarboxylate de bis(7-méthyloctyle), que l'on peut obtenir par hydrogénation de téréphtalate de bis(7-méthyloctyle) portant le numéro CAS 129951-42-4;
cyclohexane-1,4-dicarboxylate de bis(8-méthylnonyle), que l'on peut obtenir par hydrogénation de téréphtalate de bis(8-méthylnonyle) portant le numéro CAS 129951-40-2;
cyclohexanedicarboxylate de 1-[8-méthylnonyl]-4-octyle, que l'on peut obtenir par hydrogénation de téréphtalate de 1-[8-méthylnonyl]-4-octyle portant le numéro CAS 129951-39-9;
cyclohexanedicarboxylate de 1-décyl-4-octyle, que l'on peut obtenir par hydrogénation de téréphtalate de 1-décyl-4-octyle portant le numéro CAS 129951-41-3.

2. Utilisation d'un ester d'acide cyclohexane-1,3-dicarboxylique ou d'un acide cyclohexane-1,4-dicarboxylique selon la revendication 1 ou d'un mélange de deux ou de plusieurs d'entre eux comme agent amollissant dans des substances synthétiques.

3. Utilisation d'un ester d'acide cyclohexane-1,3-dicarboxylique ou d'un acide cyclohexane-1,4-dicarboxylique selon la revendication 1 ou d'un mélange de deux ou de plusieurs d'entre eux, qui, dans un essai sur des rongeurs à un dosage oral journalier de 1000 mg/kg de poids de corps de l'ester d'acide cyclohexane-1,3-dicarboxylique ou d'acide cyclohexane-1,4-dicarboxylique correspondant ou du mélange de deux ou de plusieurs d'entre eux par sonde buccale pendant la durée d'au moins 14 jours, ne donnent lieu, en comparaison des animaux témoins, ni à une augmentation significative du poids du foie après le traitement, ni à un doublement de l'activité spécifique, mesurée dans un produit d'homogénéisation du foie, de la palmitoyl-COA oxydase insensible au cyanure, comme agent amollissant pour la préparation de substances synthétiques à valoriser favorablement d'un point de vue toxicologique.
